# EUROPEAN PATENT APPLICATION

(11) **EP 4 578 436 A1**
(43) Date of publication of application: **02.07.2025**
(21) Application number: 23220535.1
(22) Date of filing: 28.12.2023
(51) Int. Cl.: A61J 3/07

(54) **HARD CAPSULE INCLUDING MULTIPLE LOCKING PARTS**

(30) Priority: 27.12.2023 KR 20230192879
(71) Applicant: Suheung Co., Ltd., Cheongju-si, Chungcheongbuk-do 28161 (KR)
(72) Inventor: YANG, Joo Hwan, 28161 Cheongjusi, Chungcheongbuk-do (KR)
(74) Representative: BCKIP Part mbB

(57) **Abstract**

The present invention is in regard to multiple specification Hard Capsule, which is formed via the joint locking between the multiple Locks of the Cap(upper part) and Body(lower part) to allow production of single hard capsules capable of functioning as variable sizes.

## Description

### BACKGROUND

### 1. Field of the Invention

The present invention relates to two-piece hard capsules that include multiple locking parts, allowing the manufacture of hard capsules with various specifications.

### 2. Discussion of Related Art

People take medicines and dietary supplements to treat diseases and improve their health.

In general, tablets and capsules are the most common types of pill and capsules can be divided into soft and hard types. A tablet is a solid form with a film coating to be dissolved and absorbed in the body. Soft capsules are mostly made from gelatin and manufactured by placing active ingredients and additives between two film sheets made up of gelatin and applying pressure to them. Therefore, the soft capsule is formed in a single piece. Hard capsules, on the other hand, are composed of a rigid shell in two pieces - a Cap and Body - and are combined and then filled with active ingredients and additives inside of capsules. The manufacture of hard capsules is easier and less expensive than that of soft capsules in general.

There are two manufacturing methods for hard capsules, cool and thermal gelation depending on gel properties. Thermal gelation is a method using HPMC or other related polymers that has a rapid viscosity increase at high temperatures. Specifically, it is a method in which a high-temperature tooling is dipped in an HPMC solution at room temperature or higher, and the solution is formed on the heated pin.

Cool gelation is a method using a gelling agent, in which a solution at room temperature is molded and then dried. In both the thermal and cool gelation methods, the solution on the mold pin tooling is dried out and the two parts of the hard capsule are stripped from the mold pin tooling. Then, Active ingredients, etc. are filled in the separate hard capsule, and the two parts are combined to produce a final product such as medicine or dietary supplement.

Meanwhile, the size of hard capsules is standardized, and the appropriate sizes are selected depending on the amount and properties of the active ingredients. However, it is difficult and very capital intensive to manufacture capsules of all of the various sizes because only one specific size of capsule can be manufactured using the existing method combining the Cap and Body of the capsule with fixed tooling in standardized sizes. Therefore, different tooling, equipment or machinery is required to produce capsules of each of the different sizes.

This invention solves two problems: 1. Due to the non homogeneous particle size in many medicine or dietary supplement fills, the density of this fill material may change as the hard capsules are being filled. This non uniformity of fill material density yields filled hard capsules which may not meet the prescribed amount of active materials per capsule. Since hard capsules are currently a fixed size, adjusting the fill material volume over the nominal capacity of the standardized fixed capsule size during the encapsulation process is impossible. A single capsule with multiple volume specifications, as this invention describes, which may be adjusted during the encapsulation process solves this problem. 2. Both the capsule manufacturer and the capsule filler must have many sets of tooling to accommodate production of the many fixed capsule sizes. A hard capsule which can be made, as this invention describes, to be adjusted to variable sizes, significantly reduces the number of tooling sets required. This reduces the overall cost throughout the production process and supply chain for medicine and dietary supplements significantly increasing their socio-economic availability.

### SUMMARY OF THE INVENTION

The present invention is intended to solve the above-mentioned problem, and it is possible to manufacture hard capsules of various sizes by combining the Cap and Body parts including multiple locking parts.

To achieve the above task, hard capsules having various specifications may be manufactured by forming multiple locking parts.

### BRIEF DESCRIPTION OF THE DRAWINGS

The above and other objects, features and advantages of the present disclosure will become more apparent to those of ordinary skill in the art by describing exemplary embodiments thereof in detail with reference to the accompanying drawings, in which:

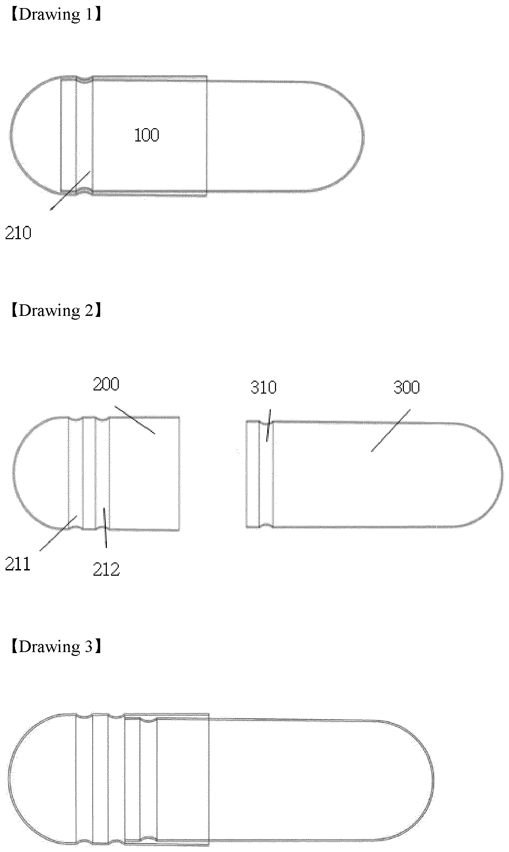

FIG. 1 illustrates a hard capsule according to the related art;
FIG. 2 illustrates a hard capsule before coupling according to an embodiment;
FIG. 3 illustrates a temporary locking state according to an embodiment;

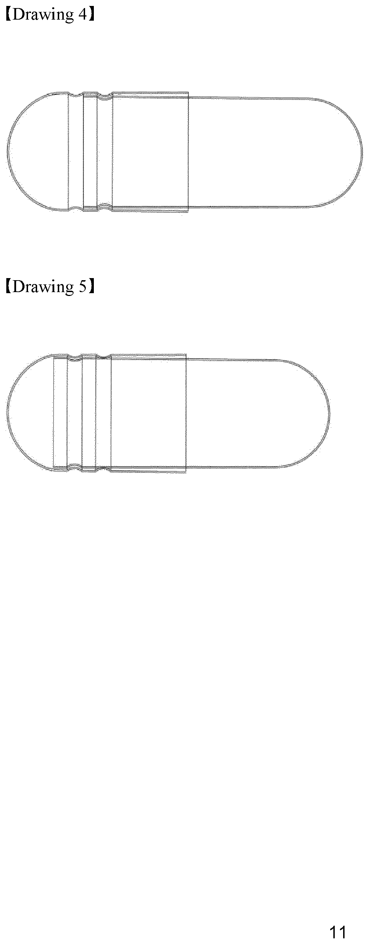

FIG. 4 illustrates a first locking state according to an embodiment; and
FIG. 5 illustrates a second locking state according to an embodiment.

### DETAILED DESCRIPTION OF EXEMPLARY EMBODIMENTS

The present disclosure may all be achieved by the following description. The following description should be understood as describing embodiments of the present invention, and the present invention is not necessarily limited thereto. Further, it should be understood that the accompanying drawings are intended to help understanding, and the present invention is not limited thereto.

Embodiments of the present invention may be variously changed and may be implemented in various forms. Thus, it should be understood that all changes, equivalents, and substitutes within the scope in which the same spirit and technical features of the present invention are recognized are included in the present disclosure.

The specifications of a hard capsule are illustrated in Table 1 below. Table 1 illustrates lengths of an upper part Cap of the hard capsule and a lower part Body of the hard capsule, a length in a temporary locking state Open L, a length in a closed state Closed L, and outer diameters of the Cap and Body of the hard capsule.

**[Table 1]**

| Size | Length (mm) | | | | Outside Diameter (mm) | |
|---|---|---|---|---|---|---|
| | Cap | Body | Open L. (Empty) | Closed L (Filled) | Cap | Body |
| #000 | 12.9 | 22.2 | 28.1 | 26.1 | 9.91 | 9.55 |
| #00EL | 13.0 | 22.2 | 27.5 | 25.3 | 8.53 | 8.18 |
| #00 | 11.7 | 20.2 | 25.5 | 23.3 | 8.53 | 8.18 |
| #0EL | 12.0 | 20.7 | 25.6 | 23.6 | 7.65 | 7.33 |
| #0 | 10.7 | 18.4 | 23.6 | 21.7 | 7.64 | 7.33 |
| #1EL | 10.5 | 17.7 | 22.4 | 20.4 | 6.91 | 6.63 |
| #1 | 9.8 | 16.6 | 21.3 | 19.4 | 6.91 | 6.63 |
| #1SL | 9.78 | 13.95 | 18.6 | 16.7 | 6.91 | 6.63 |
| #2EL | 9.7 | 16.7 | 21.0 | 19.3 | 6.35 | 6.09 |
| #2 | 8.9 | 15.3 | 19.6 | 18.0 | 6.35 | 6.07 |
| #2SL | 8.9 | 13.3 | 17.6 | 16.0 | 6.35 | 6.07 |
| #3 | 8.1 | 13.6 | 17.7 | 15.9 | 5.82 | 5.56 |
| #4 | 7.2 | 12.2 | 16.0 | 14.3 | 5.31 | 5.05 |
| #5 | 6.2 | 9.3 | 12.8 | 11.1 | 4.91 | 4.68 |

Hard Capsule (100) is comprised of the top Cap(200) and bottom Body(300), with the Cap(200) having a wider diameter. Hence the Body(300) is inserted into the Cap(200) to form Hard Capsule(100). Both Cap(200) and Body(300) each contain a locking part; Cap Lock(210) and Body Lock(310). Both locks(210, 310) are concave-shaped to prevent involuntary unlocking after closure.

The Cap (200) contains two Cap Locks (210). The length of the hard capsule is subject to change dependent on position of the part locked.

The present disclosure will be described in more detail through the following examples. However, the below examples do not limit the present disclosure in any circumstances.

### Example 1

Through creation and subsequent usage of Size #00EL/#00 Mold Pins, two locks are formed on the Cap (200) of the hard capsule. Next, the caps were produced through utilization of the production process for empty hard capsules (EMBO CAPS VG PRO by Suheung).

Example 1 shows the formation of Cap Lock #1 (211) and Cap Lock #2 (212). Example 1 shows the formation of Body Lock(310) on Body(300).

Example 1 shows Locked State #1 between Cap Lock #1 (211) and Body Lock(310). The size of Locked State #1 is the equivalent of Size #00.

Example 1 shows Locked State #2 between Cap Lock #2 (212) and Body Lock(310). The size of Locked State #2 is the equivalent of Size #00EL.

### Comparative Example 1, 2

Hard capsules of only one size were produced through utilization of the production process for empty hard capsules (EMBO CAPS VG PRO by Suheung).

Comparative Example 1 corresponds to Size #00EL, and Comparative Example 2 to Size #00.

### Comparative experiment on filling capacity of Example 1 and Comparative Examples 1 and 2.

Hard capsules of Size #00EL produced via Comparative Example 1 were cross-checked with Size #00 produced via Comparative Example 2 to compare their filling capacity. The results indicate the methods utillized for Comparative Example #1 is equal to those utilized for Comparative Example #2.

**[Table 2]**

| | | | | | |
|---|---|---|---|---|---|
| Filling Machine | | EXC-100F(Suheung) | | | |
| Speed | | 100,000 capsule/h | | | |
| Vacuum Pressure | | 20cmHg | | | |
| Size | | #00EL | | #00 | |
| Quantity | | 500,000 | | 500,000 | |
| Result | | Example 1 | Comparative Example 1 | Example 2 | Comparative Example 2 |
| | Telescope | 0 | 0 | 0 | 0 |
| | Dent | 0 | 0 | 0 | 0 |

### [List of notions explained by present invention]

100: Hard Capsule
200: Hard Capsule Cap
210: Lock of Hard Capsule Cap
211: Lock #1 of Hard Capsule Cap
212: Lock #2 of Hard Capsule Cap
300: Hard Capsule Body
310: Lock of Hard Capsule Body

## Claims

1. A hard capsule comprising a cap and a body, wherein the cap includes a first cap lock and a second cap lock.

2. The hard capsule according to claim 1, wherein the body includes a body lock, and wherein the first cap lock and the body lock are in a joint-locked state.

3. The hard capsule according to claim 1, wherein the body includes a body lock, and wherein the second cap lock and the body lock are in a joint-locked state.
